# EUROPEAN PATENT SPECIFICATION

(11) **EP 1 871 244 B1**
(45) Date of publication and mention of the grant of the patent: **03.06.2015**
(21) Application number: 06750267.4
(22) Date of filing: 14.04.2006
(51) Int. Cl.: A61B 17/22, G01N 33/50, A61L 31/00, A61B 10/02, A61B 10/04, A61B 17/3207, A61B 19/00, A61B 10/00, G06F 19/00, A61B 17/00

(54) **Compositions comprising excised vacular tissue**
Zusammensetzungen enthaltend herausgeschnittenes Gefässgewebe
Compositions comprenant de tissus vasculaire excisé

(30) Priority: 19.04.2005 US 108887
(43) Date of publication of application: 02.01.2008
(73) Proprietor: Covidien LP, Mansfield, MA 02048 (US)
(72) Inventor: SIMPSON, John, B., Woodside, California 94062 (US)
(74) Representative: Gray, James
(86) International application number: PCT/US2006/014187
(87) International publication number: WO 2006/113494

(56) References cited:
- US-A1- 2003 120 295
- US-A1- 2004 167 553
- DAVIS ET AL: "Endoscopic vein harvest for coronary artery bypass grafting: technique and outcomes" JOURNAL OF THORACIC AND CARDIOVASCULAR SURGERY, MOSBY-YEAR BOOK, INC., ST. LOUIS, MO, US, vol. 116, no. 2, August 1998 (1998-08), pages 228-235, XP005694337 ISSN: 0022-5223
- GONSCHIOR P ET AL: "Experimental directional atherectomy injury in arterial vessels: Impact of trauma depth on cellular response" AMERICAN HEART JOURNAL, MOSBY- YEAR BOOK INC., ST. LOUIS, MO, US, vol. 129, no. 6, June 1995 (1995-06), pages 1067-1077, XP004528687 ISSN: 0002-8703
- VIOLARIS A G ET AL: "Increased extracellular matrix synthesis by smooth-muscle cells obtained from in vivo restenotic lesions by directional coronary atherectomy" AMERICAN HEART JOURNAL, MOSBY- YEAR BOOK INC., ST. LOUIS, MO, US, vol. 131, no. 3, March 1996 (1996-03), pages 613-615, XP004530626 ISSN: 0002-8703
- HOSONO MITSUHARU ET AL: "Increased expression of T cell activation markers (CD25, CD26, CD40L and CD69) in atherectomy specimens of patients with unstable angina and acute myocardial infarction" ATHEROSCLEROSIS, AMSTERDAM, NL, vol. 168, no. 1, May 2003 (2003-05), pages 73-80, XP002300852 ISSN: 0021-9150
- WEISS S M ET AL: "FAILURE TO DETECT CHLAMYDIA PNEUMONIAE IN CORONARY ATHEROMAS OF PATIENTS UNDERGOING ATHERECTOMY" JOURNAL OF INFECTIOUS DISEASES, CHICAGO, IL, US, vol. 173, no. 4, 1996, pages 957-962, XP009028996 ISSN: 0022-1899
- KRINGS W ET AL: "[Ultrastructural and proliferation studies of human, catheter atherectomy extracted plaque material]" VASA. SUPPLEMENTUM 1991, vol. 33, 1991, pages 149-150, XP009078291 ISSN: 0251-1029
- DARTSCH P. C. ET AL: "Cell constitution and characteristics of human atherosclerotic plaques selectively removed by percutaneous atherectomy" ATHEROSCLEROSIS, , 80(2), 149-157, 25 REFS. ISSN: 0021-9150, 1989, XP009078282

## Description

### CROSS-REFERENCES TO RELATED APPLICATIONS

The present application is a continuation-in-part of U.S. patent application Ser. No. 10/896,741, filed Jul. 21, 2004, which is a continuation-in-part of U.S. patent application Ser. No. 10/288,559, filed Nov. 4, 2002.

### BACKGROUND OF THE INVENTION

The present invention relates generally to percutaneous transluminal systems and methods for debulking body lumens. More particularly, the present invention relates to atherectomy catheters for excising atheroma and other materials from blood vessels and from stents. The present invention also relates to methods for the selective excision and testing of material from a body lumen, such as a blood vessel.

Cardiovascular disease frequently arises from the accumulation of atheromatous material on the inner walls of vascular lumens, particularly arterial lumens of the coronary and other vasculature, resulting in a condition known as atherosclerosis. Atherosclerosis occurs naturally as a result of aging, but may also be aggravated by factors such as diet, hypertension, heredity, vascular injury, and the like. Atheromatous and other vascular deposits restrict blood flow and can cause ischemia which, in acute cases, can result in myocardial infarction. Atheromatous deposits can have widely varying properties, with some deposits being relatively soft and others being fibrous and/or calcified. In the latter case, the deposits are frequently referred to as plaque.

One conventional treatment for cardiovascular disease is the use of stents. Endoluminal stents are commonly used to treat obstructed or weakened body lumens, such as blood vessels and other vascular lumens. Once deployed in the blood vessel, the stent can remain in the body lumen where it will maintain the patency of the lumen and/or support the walls of the lumen which surround it. One factor impeding the success of stent technology in endoluminal treatments is the frequent occurrence of in-stent restenosis, characterized by proliferation and migration of smooth muscle cells within and/or adjacent to the implanted stent, causing reclosure or blockage of the body lumen.

Atherosclerosis and restenosis can be treated in a variety of ways, including drugs, bypass surgery, and a variety of catheter-based approaches which rely on intravascular debulking or removal of the atheromatous or other material occluding a blood vessel. Of particular interest to the present invention, a variety of methods for cutting or dislodging material and removing such material from the blood vessel have been proposed, generally being referred to as atherectomy procedures. Atherectomy catheters intended to excise material from the blood vessel lumen generally employ a rotatable and/or axially translatable cutting blade which can be advanced into or past the occlusive material in order to cut and separate such material from the blood vessel lumen. In particular, side-cutting atherectomy catheters generally employ a housing having an aperture on one side, a blade which is rotated or translated by the aperture, and a balloon to urge the aperture against the material to be removed.

Although atherectomy catheters have proven very successful in treating many types of atherosclerosis and in-stent restenosis, improved atherectomy catheters and methods are continuously being pursued. For example, many currently available side-cutting atherectomy catheters have difficulty in capturing occluding material in the cutting aperture. To facilitate material capture, the cutting aperture is frequently elongated to increase the area into which the material can penetrate. Such elongation typically requires an equivalent lengthening of the cutter housing. Since most cutter housings are rigid, such lengthening makes it more difficult to introduce the distal end of the catheter through tortuous regions of the vasculature.

Another shortcoming of many currently available atherectomy catheters is that they typically require a balloon positioned opposite the cutting window to urge the cutting window into contact with occluding material. Such balloons, however, unduly increase the size of the distal portion of the catheter. Even with the balloon, the amount of material that can be removed by conventional atherectomy catheters is limited by the size of the cutting window. Other disadvantages of some catheters include cutting elements with less than ideal hardness, inadequate storage space within the catheter for containing removed material, sub-optimal guide wire lumens, and/or the like. In addition, the available atherectomy catheters generally provide material insufficient in quantity and/or quality for testing by many histological, array, proteomic or other biochemical or molecular methods. For example, in one report a device and method available to the artisan collected less than about 50 mg of tissue. (Safian et al., Circulation 82:305-307 (1990)). This amount of material is not typically enough to carry out more than one test, or is insufficient to successfully carry out a number of diagnostic tests available to the physician or researcher.

For these reasons, it would be advantageous to have atherectomy catheters which could access small, tortuous regions of the vasculature and remove atheromatous and other occluding materials from within blood vessels and stents in a controlled fashion. In particular, it would be desirable to have atherectomy catheters which could facilitate capturing and invagination of atheromatous materials. Particularly, those capable of in vivo capturing and removing at least one continuous tissue strand of sufficient quantity and quality for testing in vitro. Ideally, such catheters and methods for their use would be adaptable for use in a variety of body lumens, including but not limited to coronary and other arteries. At least some of these objectives will be met by the present invention.

### BRIEF SUMMARY OF THE INVENTION

According to a first aspect of the present invention, there is provided a composition of excised vascular tissue comprising at least one continuous strand of tissue that has been removed from along the axis of the inner surface of a vascular lumen in a human body and preserved, wherein the continuous strand comprises a length of at least 2 cm, a depth of at least 0.1 mm and a volume of at least 0.45 mg/mm of length.

The composition may comprise a plurality of continuous strands.

The plurality of strands may be collected from a single vascular lumen or a single vascular obstruction.

The continuous strand may have a length selected from the group consisting of 5cm or greater in length, 7cm or greater in length, 10cm or greater in length, and 15cm or greater in length.

The continuous strand may have a depth selected from the group consisting of at least 0.25 mm, at least 0.33 mm, and at least 0.5 mm, wherein depth is a dimension which is radial to the axis of the lumen.

The continuous strand may have a volume selected from the group consisting of at least 0.50 mg/mm of length, at least 0.55 mg/mm of length, at least 0.60 mg/mm of length, at least 0.65 mg/mm of length, and at least 0.70 mg/mm of length.

According to a further aspect of the present invention, there is provided a library comprising a plurality of samples of excised vascular tissue according to the first aspect.

The samples may be preserved so as to maintain the continuous strand intact.

The samples may be maintained in a preserving agent, tissue fixative, or test preparation agent selected from the group consisting of saline, heparinized saline, liquid nitrogen, formalin, a membrane lysis agent, an RNA preparation agent, and a DNA preparation agent.

Clinical information regarding patients from whom the samples were excised may be maintained with the library.

According to a further aspect of the present invention, there is provided a library comprising a plurality of samples of material derived from excised vascular tissue according to the first aspect, wherein the plurality of samples of derived material are selected from the group consisting of DNA, RNA, cDNA, and protein.

The clinical information regarding patients from whom the samples were excised may be maintained with the library.

According to a further aspect of the present invention, there is provided an ex vivo method of gathering information about vascular disease in an individual or a population of individuals comprising providing excised vascular tissue removed from a body lumen of one or more individuals wherein the tissue comprises a composition of the first aspect, performing one or more tests on at least a portion of the excised and removed vascular tissue composition, and recording the results of the tests for use in a process selected from the group consisting of identifying, diagnosing, treating, and researching vascular disease.

The tests may comprise a test selected from the group consisting of genomic screening, DNA hybridization, RNA hybridization, gene expression analysis, PCR amplification, proteomic testing, drug efficacy screening, determining the presence of one or more DNA markers, determining the presence of one or more RNA markers, determining the presence of one or more protein markers, histological screening, histopathology, cytopathology, cell type analysis, tissue type analysis, and biopsy.

The test may comprise analysing the vascular tissue composition for the presence of DNA, RNA, or a protein marker comprising a moiety selected from the group consisting of a smooth muscle proliferative promoter, a smooth muscle proliferative inhibitor, a cellular marker, an apoptotic marker, a cell cycle protein, a transcriptional factor, a proliferative marker, an endothelial growth factor, an adhesion molecule, a cytokine, a chemokine receptor, an inflammation marker, an extracellular matrix molecule, an interleukin, a growth factor, a glycoprotein, a proteoglycan, a cell-surface marker, a serum marker, and an immune factor.

The vascular tissue may have been excised and removed from more than one site in a single vascular lumen of an individual.

The vascular tissue may have been excised and removed from more than one vascular lumen of an individual.

The vascular tissue excised and removed from an individual may comprise from 1 mg to 2000 mg of vascular tissue.

The vascular tissue excised and removed from a population of individuals may comprise on average from 1 mg to 2000 mg of vascular tissue from each individual.

The steps of providing excised vascular tissue may comprise providing a library.

The method may further comprise providing a plurality of samples of material derived from vascular tissue selected from the group consisting of DNA, RNA, cDNA, and protein.

For a further understanding of the nature and advantages of the invention, reference should be made to the following description taken in conjunction with the accompanying drawings.

### BRIEF DESCRIPTION OF THE DRAWINGS

FIG. 1 shows the weight and lengths of 20 fragments of vascular material excised according to the present invention.

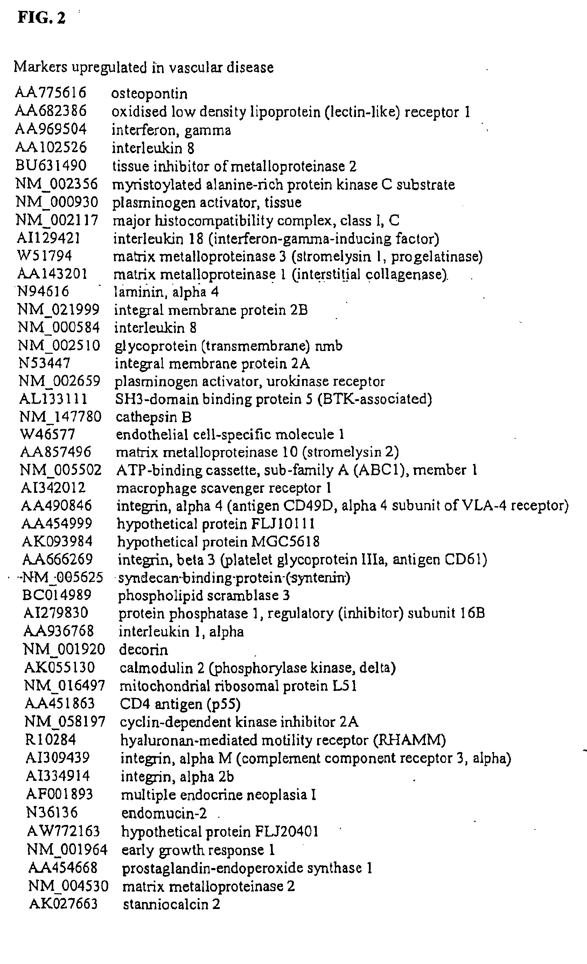

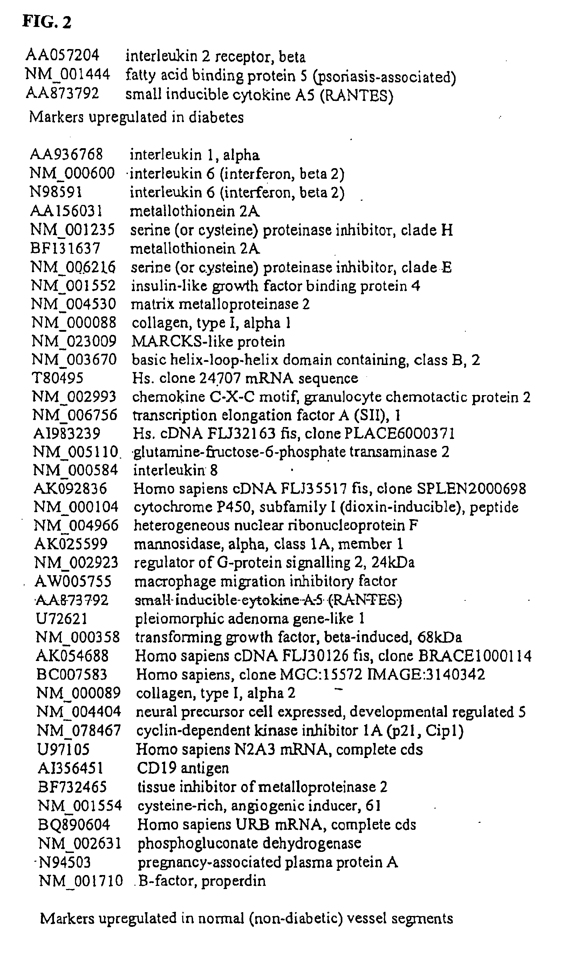

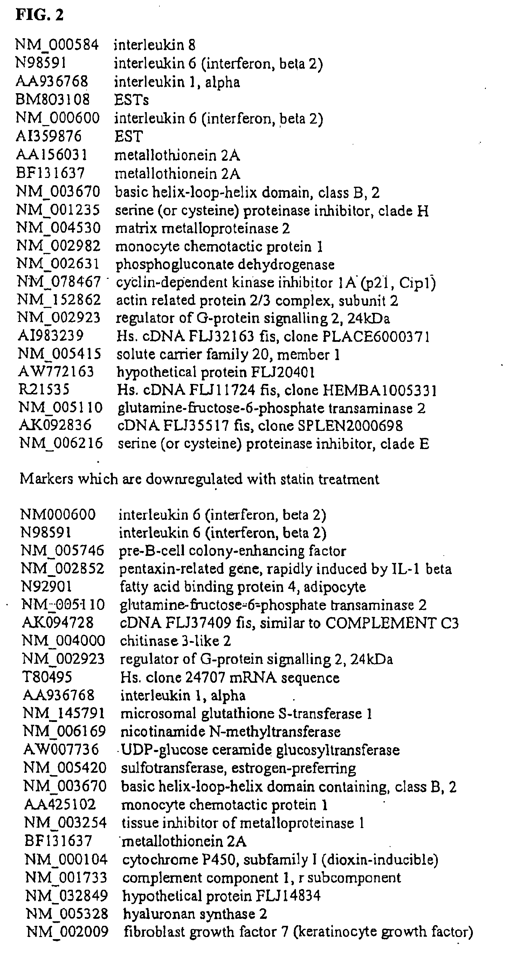

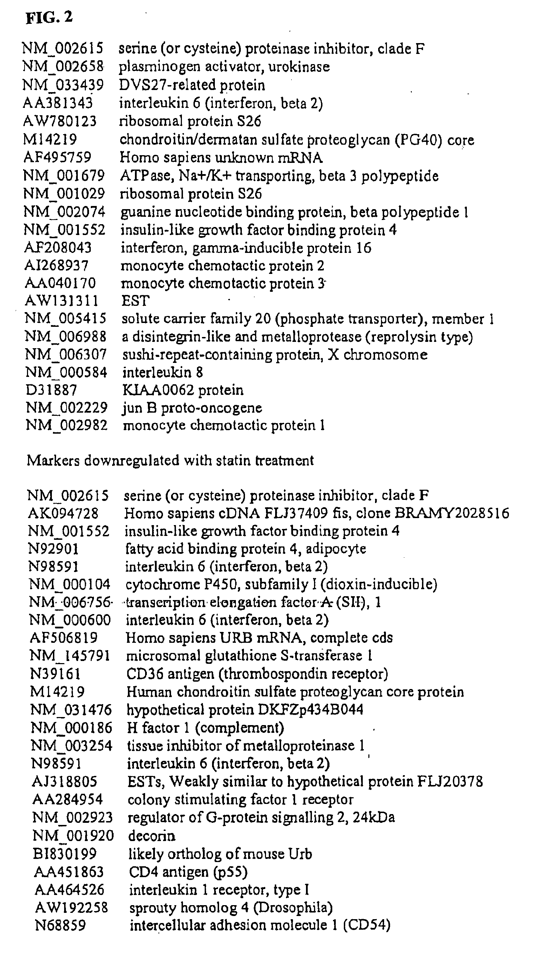

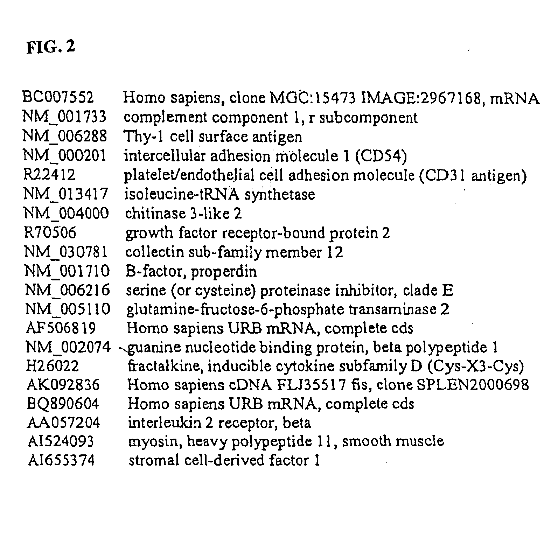

FIG. 2 shows relevant markers for which the excised vascular material can be tested for expression.

### DETAILED DESCRIPTION OF THE INVENTION

The catheters and methods of the present invention are designed to debulk atheroma and other occlusive material from diseased body lumens, and in particular coronary arteries, de novo lesions, and in-stent restenosis lesions. The catheters and methods, however, are also suitable for treating stenoses of body lumens and other hyperplastic and neoplastic conditions in other body lumens, such as the ureter, the biliary duct, respiratory passages, the pancreatic duct, the lymphatic duct, and the like. Neoplastic cell growth will often occur as a result of a tumor surrounding and intruding into a body lumen. Debulking of such material can thus be beneficial to maintain patency of the body lumen. The catheters and methods of the present invention also provide methods that provide lumenectomy samples or materials that are of higher quality and quantity that typically have been provided by prior devices. The material provided is typically a continuous strip of tissue removed from the lumen interior wall that ranges from about 1 mg to about 2000 mg and wherein the tissue retains the structure of the tissue prior to removal. Advantageously, the continuous strip or strand of tissue removed will typically have a length that is longer than a length of the cutting window. While the remaining discussion is directed at debulking and passing through atheromatous or thrombotic occlusive material in a coronary artery, it will be appreciated that the systems and methods of the present invention can be used to remove and/or pass through a variety of occlusive, stenotic, or hyperplastic material in a variety of body lumens.

Apparatus according to the present invention will generally comprise catheters having catheter bodies adapted for intraluminal introduction to the target body lumen. The dimensions and other physical characteristics of the catheter bodies will vary significantly depending on the body lumen which is to be accessed. In the exemplary case of atherectomy catheters intended for intravascular introduction, the proximal portions of the catheter bodies will typically be very flexible and suitable for introduction over a guidewire to a target site within the vasculature. In particular, catheters can be intended for "over-the-wire" introduction when a guidewire channel extends fully through the catheter body or for "rapid exchange" introduction where the guidewire channel extends only through a distal portion of the catheter body. In other cases, it may be possible to provide a fixed or integral coil tip or guidewire tip on the distal portion of the catheter or even dispense with the guidewire entirely. For convenience of illustration, guidewires will not be shown in all embodiments, but it should be appreciated that they can be incorporated into any of these embodiments.

Catheter bodies intended for intravascular introduction will typically have a length in the range from 50 cm to 200 cm and an outer diameter in the range from 1 French to 12 French (0.33 mm: 1 French), usually from 3 French to 9 French. In the case of coronary catheters, the length is typically in the range from 125 cm to 200 cm, the diameter is preferably below 8 French, more preferably below 7 French, and most preferably in the range from 2 French to 7 French. Catheter bodies will typically be composed of an organic polymer which is fabricated by conventional extrusion techniques. Suitable polymers include polyvinylchloride, polyurethanes, polyesters, polytetrafluoroethylenes (PTFE), silicone rubbers, natural rubbers, and the like. Optionally, the catheter body may be reinforced with braid, helical wires, coils, axial filaments, or the like, in order to increase rotational strength, column strength, toughness, pushability, and the like. Suitable catheter bodies may be formed by extrusion, with one or more channels being provided when desired. The catheter diameter can be modified by heat expansion and shrinkage using conventional techniques. The resulting catheters will thus be suitable for introduction to the vascular system, often the coronary arteries, by conventional techniques.

The distal portion of the catheters of the present invention may have a wide variety of forms and structures. In many embodiments, a distal portion of the catheter is more rigid than a proximal portion, but in other embodiments the distal portion may be equally as flexible as the proximal portion. One aspect of the present invention provides catheters having a distal portion with a reduced rigid length. The reduced rigid length can allow the catheters to access and treat tortuous vessels and small diameter body lumens. In most embodiments a rigid distal portion or housing of the catheter body will have a diameter that generally matches the proximal portion of the catheter body, however, in other embodiments, the distal portion may be larger or smaller than the flexible portion of the catheter.

A rigid distal portion of a catheter body can be formed from materials which are rigid or which have very low flexibilities, such as metals, hard plastics, composite materials, NiTi, steel with a coating such as titanium nitride, tantalum, ME-92®, diamonds, or the like. Most usually, the distal end of the catheter body will be formed from stainless steel or platinum/iridium. The length of the rigid distal portion may vary widely, typically being in the range from 5 mm to 35 mm, more usually from 10 mm to 25 mm, and preferably between 6 mm and 8 mm. In contrast, conventional catheters typically have rigid lengths of approximately 16 mm.

The side opening windows of the present invention will typically have a length of approximately 2 mm. In other embodiments, however, the side opening cutting window can be larger or smaller, but should be large enough to allow the cutter to protrude a predetermined distance that is sufficient to debulk material from the body lumen.

The catheters of the present invention can include a flexible atraumatic distal tip coupled to the rigid distal portion of the catheter. For example, an integrated distal tip can increase the safety of the catheter by eliminating the joint between the distal tip and the catheter body. The integral tip can provide a smoother inner diameter for ease of tissue movement into a collection chamber in the tip. During manufacturing, the transition from the housing to the flexible distal tip can be finished with a polymer laminate over the material housing. No weld, crimp, or screw joint is usually required.

The atraumatic distal tip permits advancing the catheter distally through the blood vessel or other body lumen while reducing any damage caused to the body lumen by the catheter. Typically, the distal tip will have a guidewire channel to permit the catheter to be guided to the target lesion over a guidewire. In some exemplary configurations, the atraumatic distal tip comprises a coil. In some configurations the distal tip has a rounded, blunt distal end. The catheter body can be tubular and have a forward-facing circular aperture which communicates with the atraumatic tip. A collection chamber can be housed within the distal tip to store material removed from the body lumen. The combination of the rigid distal end and the flexible distal tip is approximately 30 mm.

A rotatable cutter or other tissue debulking assembly may be disposed in the distal portion of the catheter to sever material which is adjacent to or received within the cutting window. In an exemplary embodiment, the cutter is movably disposed in the distal portion of the catheter body and movable across a side opening window. A straight or serrated cutting blade or other element can be formed integrally along a distal or proximal edge of the cutting window to assist in severing material from the body lumen. In one particular embodiment, the cutter has a diameter of approximately 1.14 mm. It should be appreciated however, that the diameter of the cutter will depend primarily on the diameter of the distal portion of the catheter body.

In exemplary embodiments, activation of an input device can deflect a distal portion of the catheter relative to the proximal portion of the catheter. Angular deflection of the distal portion may serve one or more purposes in various embodiments. Generally, for example, deflection of the distal portion increases the effective "diameter" of the catheter and causes the debulking assembly to be urged against material in a lumen, such as, but not limited to, atherosclerotic plaque. In other embodiments, deflection of the distal portion may act to expose a debulking assembly through a window for contacting material in a lumen. In some embodiments, for example, activation of the input device moves the debulking assembly over a ramp or cam so that a portion of the rigid distal portion and flexible tip are caused to drop out of the path of the debulking assembly so as to expose the debulking assembly through the window. In some embodiments, deflection may both urge a portion of the catheter into material in a lumen and expose a tissue debulking assembly.

It should be understood movement of a tissue debulking assembly may cause deflection of a portion of the catheter or that deflection of the catheter may cause movement or exposure of a tissue debulking assembly, in various embodiments. In other embodiments, deflection of a portion of the catheter and movement of the tissue debulking assembly may be causally unconnected events. Any suitable combination of deflecting, exposing of a debulking assembly and the like is contemplated. In carrying out deflection, exposure and/or the like, a single input device may be used, so that a user may, for example, deflect a portion of a catheter and expose a tissue debulking assembly using a single input device operable by one hand. In other embodiments, rotation of a tissue debulking assembly may also be activated by the same, single input device. In other embodiments, multiple input devices may be used.

Some embodiments further help to urge the debulking assembly into contact with target tissue by including a proximal portion of the catheter body having a rigid, shaped or deformable portion. For example, some embodiments include a proximal portion with a bend that urges the debulking assembly toward a side of the lumen to be debulked. In other embodiments, one side of the proximal portion is less rigid than the other side. Thus, when tension is placed on the catheter in a proximal direction (as when pulling the debulking assembly proximally for use), one side of the proximal portion collapses more than the other, causing the catheter body to bend and the debulking assembly to move toward a side of the lumen to be debulked.

In exemplary embodiments, the debulking assembly comprises a rotatable cutter that is movable outside the window. By moving the cutter outside of the cutting window beyond an outer diameter of the distal portion of the catheter, the cutter is able to contact and sever material that does not invaginate into the cutting window. In a specific configuration, the rotating cutter can be moved over the cam within the rigid, or distal, portion of the catheter body so that the cutting edge is moved out of the window. Moving the rotating cutter outside of the cutting window and advancing the entire catheter body distally, a large amount of occlusive material can be removed. Consequently, the amount of material that can be removed is not limited by the size of the cutting window.

Certain embodiments of the present invention provide for methods for in vivo excising and removing material from the inner wall of one or more lumen that is of higher quantity and quality than prior devices or methods. The material removed therefore is better suited for use in various testing methods. Particularly, the methods provide sufficient material or better quality and quantity for use in one or more tests from a single percutaneous translumenal lumenectomy procedure. Further, the material typically maintains the structure possessed by the material in vivo. This provides for the ability to carry out certain tests, such as histology, cytopathology, and other tests that have been difficult to perform using prior devices and methods.

In one embodiment the method of the present invention for excising and testing material from a body lumen comprises the steps of providing a catheter having a rotating cutter, a collection chamber, and a cutting window, the rotating cutter being movable between a stored position and an exposed position, at least part of the rotating cutter becoming exposed through the cutting widow when moving to the exposed position. The catheter is advanced transluminally through the body lumen to move or plane the rotating cutter through material in a first site in the body lumen, the rotating cutter remaining in the exposed position so that the cutter and the widow maintain their orientation with respect to one another when advancing the catheter and planing through the material. The planing action of the present invention provides a substantially consistent and even tissue removal through the body lumen. The continuous strand of material cut by the rotating cutter is directed through the cutting widow and into the collection chamber as the catheter is advanced through the material. The material can then be removed from the collection chamber and one or more tests performed on at least a portion of the material removed from the collection chamber.

The material or tissue excised from the body lumen will vary in length and will depend on the catheter configuration, the type of material removed, the body lumen, and the like. However, in certain embodiments, the material will be in the form of continuous strands that has a substantially consistent depth and width of tissue cuts. The material is typically longer than the length of the cutting window (but it may be shorter), and typically has a length of about 2.0 mm or longer, and sometimes between about 0.5 cm up to about 10 cm or longer in length. Typically the length of a continuous strand is at least 2 cm, at least 5 cm, at least 7 cm, at least 10 cm, or at least 15 cm. The length of a strand is the dimension which is axial to the lumen. Advantageously, the planing action of the catheter provides a material tissue structure that reflects the actual in vivo tissue structure, and provides information about larger portions of the disease state of the body lumen. One or more strands may be obtained from a single vascular lumen or single vascular obstruction. Because of the design and configuration of the device, the strands typically have a depth of at least 0.1 mm, at least 0.25 mm, at least 0.33 mm, or at least 0.5 mm. Depth of a strand is the dimension which is radial to the axis of a lumen. The cutting and planing action of the device of the invention achieves large volumes which are excellent for analysis, for multiple analyses, for storage as archival samples, and for assembly into libraries of samples representative of certain disease states. The mass/length ratio of continuous strands is typically at least 0.45 mg/mm, at least 0.50 mg/mm, at least 0.55 mg/mm, at least 0.60 mg/mm, at least 0.65 mg/mm, or at least 0.70 mg/mm. Libraries of samples can be assembled for studies of drugs, candidate drugs, toxins, therapeutic treatments, etc. The samples can be preserved according to any method known in the art. Samples may be frozen, for example, in liquid nitrogen, they may be preserved in paraffin, dried, freeze dried, etc. Samples may be treated to achieve a purified or semi-purified component of the sample. Samples may be treated, for example to extract DNA or protein. Samples may be treated to extract mRNA and to preserve it or "convert" it to cDNA. Desirably, samples are stored in a systematic way so that patient information remains associated with the samples and patient outcome can be associated with the sample concurrently or at a later time.

Tissue retrieved via the present invention provides an opportunity for greater confidence in test results for single or multiple tests due to reduced sampling errors resulting from greater tissue volume and enhanced tissue quality than was previously possible. Previously, the retrieved atherectomized tissue samples had problems because there was only angiographic control possible for the evaluation of how much stenotic tissue has been sampled. With the present invention, it is more confidently known how much stenotic tissue is sampled with a retrieval. Generally, the smooth muscle cells of the stenotic material show a range of phenotypes, but most of the cells contained myofilaments as well as a relatively high amount of synthetic organelles, such as rough endoplasmic reticulum, Golgi apparatus and mitochondria. A larger tissue sample of better quality and more confidence in the retrieval location can help determine with confidence how much stenotic tissue is retrieved in the procedure. Because the absence of inflammatory cells in excised tissue may be only one of many variables in sorting out a cardiovascular condition, the presence of the inflammatory cells within critical areas of plaque may be more important than their sheer number. Using the methods of the present invention, the location and degree of inflammatory cells present may be determined in order to facilitate a more informed diagnosis.

In the past, there has been difficulty obtaining sufficient plaque or other material to perform proliferation studies and to sub-cultivate tissue. The problem associated with the inability to obtain fresh human plaque tissue is underscored by the significant need for a tissue model wherein smooth muscle cells can be analyzed with as many of the original characteristics attributed to the previous in vivo injury as possible. Sufficient tissue of good quality will obviate the need to cultivate these cells. To date it has not been possible to directly compare smooth muscle cells from restenotic and primary lesions, a comparison that could be facilitated using primary and restenotic tissue retrieved by the present invention.

The material removed from the collection chamber, or a portion thereof, can be placed in a preserving agent, a tissue fixative, and or a preparation agent suitable for a desired test prior to testing the material. The material removed from the patient by this method is typically at least one or more continuous strip(s) of material that maintains the structure of the material in vivo. The quantity of material removed by the method can be from about 1 mg to about 2000 mg. Typically the amount of material is about 1 mg to about 100 mg, about 100 mg to about 200 mg, about 200 mg to about 300 mg, 300 mg to about 400 mg, 400 mg to about 500 mg, 500 mg to about 600 mg, about 600 mg to about 700 mg, 700 mg to about 800 mg, or about 800 mg to about 2000 mg. In a typical procedure about 400 mg to about 600 mg of material is removed and available for testing and/or storage. A preferred embodiment of the present invention provides for the collection of one or more continuous strips of material from the inner surface of the lumen that is longer than a largest dimension of the cutting window. In a particular example, the material can comprise plaque tissue.

The methods of the present invention provide high quality material is a sufficient quantity that allows for multiple testing methods (e.g., validation testing, repeat testing, etc.) and provides a sufficient amount of sample to allow storage of an amount of sample to allow later confirmatory or additional testing to confirm a diagnosis without having to subject the patient to another percutaneous translumenal lumenectomy procedure. The methods can provide sufficient high quality material for tests comprising genomic screening, DNA hybridization, RNA hybridization, gene expression analysis, PCR amplification, proteomic testing, drug efficacy screening, a presence of one or more protein markers, a presence of one or more DNA markers, a presence of one or more RNA markers, histological testing, histopathology, cytopathology, cell and tissue type analysis, biopsy, and the like. Additionally, the material can also be cultured to determine reactivity to drugs, e.g., therapeutic drugs, and the like. Being able to carry out such testing provides for the ability to perform one or more tests comprising, for example, but not limitation, analyzing the material for the presence of DNA, RNA, or a protein marker comprising a smooth muscle proliferative promoter, a smooth muscle proliferative inhibitor, a cellular marker, an apoptotic marker, a cell cycle protein, a transcriptional factor, a proliferative marker, an endothelial growth factor, an adhesion molecule, a cytokine, a chemokine, a chemokine receptor, an inflammation marker, a coagulation factor, a fibrinolytic factor, an oxidative stress related molecule, an extracellular matrix molecule, an interleukin, a growth factor, a glycoprotein, a proteoglycan, a cell-surface marker, a serum marker, and or an immune factor, and the like. Tests for each of these molecules and others are well known to the skilled artisan as are methods and preservatives, fixatives and preparation agents for adding to all or a portion of the material collected.

In another embodiment of the present invention the method can further comprise i) moving the cutter to the stored position, ii) repositioning the catheter at a second site, iii) exposing the cutter by moving the cutter to the exposed position, and iv) advancing the catheter to move the rotating cutter through material in the second site, the rotating cutter remaining in the exposed position so that the cutter and the widow maintain their orientation with respect to one another when advancing the catheter through the material, the material cut by the rotating cutter being directed through the cutting widow and into the collection chamber as the catheter is advanced through the material. The first and second sites can be in either the same or a different body lumen.

Another embodiment of the methods of the present invention for removing material from a vascular location comprises the steps of providing a catheter having a body, an opening leading to a collection chamber, and a cutter, the cutter being movable between a stored position and an exposed position, the cutter becoming at least partially exposed when moving from the stored position to the exposed position. The catheter is then percutaneously introduced into and transluminally advanced through a patient's vascular system with the cutter in the stored position, the catheter being introduced into the vascular location where material is to be removed. The cutter is then exposed by moving the cutter to the exposed position and the cutter is rotated. The catheter is then advanced after the exposing step and during the rotating step, wherein the rotating cutter and the opening advance together so that material cut by the rotating cutter is directed through the opening and into the collection chamber as the catheter is advanced. Subsequent to excising the material the catheter is removed from the vascular location and the material collected in the collection chamber is harvested and one or more tests on at least a portion of the material removed from the collection chamber can be carried out.

The material removed from the collection chamber, or a portion thereof, can be placed in a preserving agent, a tissue fixative, and or a preparation agent suitable for a desired test prior to testing the material. The material removed from the patient by this method is typically at least one or more continuous strip(s) of material that maintains the structure of the material in vivo. The quantity of material removed by the method can be from about 1 mg to about 2000 mg. Typically the amount of material is about 1 mg to about 100 mg, about 100 mg to about 200 mg, about 200 mg to about 300 mg, 300 mg to about 400 mg, 400 mg to about 500 mg, 500 mg to about 600 mg, about 600 mg to about 700 mg, 700 mg to about 800 mg, or about 800 mg to about 2000 mg. In a typical procedure about 400 mg to about 600 mg of material is removed and available for testing and/or storage. A preferred embodiment of the present invention provides for the collection of one or more continuous strips of material from the inner surface of the lumen that is longer than a largest dimension of the cutting window. In a particular example, the material can comprise plaque tissue. The material can be collected from a single site or at least one additional site in the same or a different body lumen.

The material produced by the methods of the invention provide a lumenectomy composition comprising at least one continuous tissue stand collected in vivo from an inner surface of the body lumen of a subject. In one embodiment the body lumen can be an artery or other lumen or vessel of the circulatory system and the material can comprise arterial plaque and associated tissue. The continuous stand of tissue provided by the disclosed methods provide a sufficient amount of high quality material to successfully perform at least one or more tests comprising, for example, genomic screening, DNA hybridization, RNA hybridization, gene expression analysis (including serial analysis of gene expression), PCR amplification, proteomic testing, drug efficacy screening, a determination of the presence of one or more protein markers, a determination of the presence of one or more DNA markers, a determination of the presence of one or more RNA markers, histological testing, histopathology, cytopathology, cell type analysis, tissue type analysis, biopsy, and the like. Methods for performing each of the tests are well known to the skilled artisan. It is also well known that material collected from a patient can be added to a preserving agent, tissue fixative, or a preparation agent in order to prepare at least a portion of collected material for the desired test. Agents known in the art for preserving, fixing or preparing the material for later use include, for example, saline, heparinized saline, liquid nitrogen, formalin, a membrane lysis agent, a RNA or DNA preparation agent, and the like. Particular testes that can be carried our successfully on the excised lumenectomy material removed by the methods of the present invention include, but are not limited to, histology techniques including hematoxylin and eosin staining, connective tissue staining, carbohydrate staining, and lipid staining, and the like. In addition, tissue array testing, enzyme histochemistry, transmission electron microscopy, immunohistology, immunocytochemistry, immunoassays, immunofluorescent assays, immunoprecipitation assays, ELISA, flow cytometry, fluorescent activated cell sorting, radioimmunochemistry, electrophoresis, two-dimensional gel electrophoresis, Western blotting, protein sequencing, mass spectrometry, proteomic analysis, and protein microarray analysis can be carried out. Further, cytogenetic testing, Nothern blotting, RNase protection assays, in situ hybridization assays, DNA microarray testing, reverse transcription polymerase chain reaction PCR (RT-PCR), Southern blotting, DNA sequencing, PCR amplification, single strand conformational polymorphism assays, single strand polymorphism (SNP) assays, and serial analysis of gene expression (SAGE) assays can be successfully carried out with the lumenectomy materials compositions collected by the disclosed methods. The compositions of the present invention or portions thereof can also be prepared for storage for later testing.

In certain embodiments of the present invention the material collected can be analyzed for the presence of DNA, RNA, or protein markers comprising smooth muscle proliferative promoters (platelet-derived growth factor (PDGF), and PDGF receptor), basic fibroblast growth factor (FGF) and FGF receptor, interleukin 1 (IL-1), or transforming growth factor (TGFα), and the like), smooth muscle proliferative inhibitors (nitric oxide/endothelial-derived relaxing factors (NO/EDRF), interferon γ (IFγ), transforming growth factor (TGFβ), or TGFβ receptor, and the like), cellular markers (including CD68, CD3, CD4, CD8, CD20, smooth muscle actin, or CD31, and the like), apoptotic markers (Bcl-x, Bcl-2, Bax, Bak, or P53, and the like), cell cycle proteins (cyclin A, cyclin B, cyclin D, or cyclin E, and the like), transcriptional factors (transcription factor NFκB, transcription factor E2F, transcription factor CREB, or transcription factor KLF5/BTEB2, and the like), proliferative markers (Ki-67 or proliferating cell nuclear antigen (PCNA), and the like), endothelial growth factors (vascular endothelial growth factor (VEGF), and the like), adhesion molecules (intercellular adhesion molecule-1 (ICAM-1), CD11a/CD18 (LFA-1), CD11b/CD18 (MAC-1), vascular cell adhesion molecule-1 (VCAM-1), p-selectin (CD62P), or integrin, and the like), cytokines (interleukin 6 (IL-6) or interleukin 8 (IL-8), and the like), chemokines and chemokine receptors (monocyte chemoattractant protein 1 (MCP-1) and its receptor CCR2, CX3C chemokine fractalkine and its receptor CX3CR1, or eotaxin and its receptor CCR3, and the like), inflammation markers (C-reactive protein, myeloperoxidase, or complement proteins, and the like), coagulation factors and fibrinolytic factors (fibrinogen, prothrombinogen, plasminogen activator, tissue factor, or glycoprotein receptor on platelets (GpIIb-IIIa), and the like), oxidative stress related molecules (oxidized LDL and its receptor CD36, or lipoxygenase, and the like), extracellular matrix molecules (collagen, matrix metalloproteinase (MMP), FK506-binding protein 12 (FKBP12), endothelial differentiation gene receptors (EDG receptors), ephrins, elastin, lamin receptor, monocyte colony stimulating factor (M-CSF), tumor necrosis factor (TNF), or PDZ domain proteins, and the like), interleukins (interleukin 1 (IL-1), interleukin 6 (IL-6), or interleukin 8 (IL-8), and the like), growth factors (platelet-derived frowth factor (PDGF), basic fibroblast growth factor (FGF), transforming growth factor α (TGFα), or transforming growth factor β (TGFβ), and the like), glycoproteins, proteoglycans (versican, hyluronan, biglycan, or deorin, and the like), cell-surface markers, serum markers, and/or immune factors (stromal cell-derived factor 1a (SDF-1)), and the like). Analysis of the excised material by any of the above tests can be used for diagnosis of a condition in a patient, design a treatment directive or protocol for a subject, monitor progress of a treatment regimen, or if tests from a number of individuals are compared, the information can be used in a multi-patient analysis, such as a cardiovascular disease population study.

As will be described in detail below, in some situations it is preferable to provide a serrated cutting edge, while in other situations it may be preferable to provide a smooth cutting edge. Optionally, the cutting edge of either or both the blades may be hardened, e.g., by application of a coating. A preferred coating material is a chromium based material, available from ME-92, Inc., which may be applied according to manufacturer's instructions. In some embodiments, the cutter includes a tungsten carbide cutting edge. Other rotatable and axially movable cutting blades are described in U.S. Pat. Nos. 5,674,232; 5,242,460; 5,312,425; 5,431,673; and 4,771,774. In some embodiments, a rotatable cutter includes a beveled edge for removal of material from a body lumen while preventing injury to the lumen. In still other embodiments, a tissue debulking assembly may include alternative or additional features for debulking a lumen. For example, the debulking assembly may include, but is not limited to, a radio frequency device, an abrasion device, a laser cutter and/or the like.

The catheters of the present invention may include a monorail delivery system to assist in positioning the cutter at the target site. For example, the tip of the catheter can include lumen(s) that are sized to receive a conventional guidewire (typically 0.356mm (0.014") diameter) or any other suitable guidewire (e.g., having diameters between 0.457mm (0.018") and 0.813mm (0.032")) and the flexible proximal portion of the catheter body can include a short lumen (e.g., about 12 centimeters in length). Such a configuration moves the guidewire out of the rigid portion so as to not interfere with the debulking assembly.

In other embodiments, however, the guidewire lumen may be disposed within or outside the flexible proximal portion of the catheter body and run a longer or shorter length, and in fact may run the entire length of the flexible portion of the catheter body. The guidewire can be disposed within lumen on the flexible portion of the catheter body and exit the lumen at a point proximal to the rigid portion of the catheter. The guidewire can then enter a proximal opening in the tip lumen and exit a distal opening in the tip lumen. In some embodiments, the catheter has a distal guidewire lumen on its flexible distal tip and a proximal guidewire lumen on its flexible body. For example, in some embodiments the distal lumen may have a length of between about 2.0 cm and about 3.0 cm and the proximal lumen may have a length of between about 10 cm and about 14 cm. In yet further embodiments, a distal tip guidewire lumen may be configured to telescope within a proximal guidewire lumen, or vice versa. A telescoping guidewire lumen may enhance performance of the catheter by preventing a guidewire from being exposed within a body lumen.

The present invention may optionally employ any of a wide variety of conventional radiopaque markers, imaging devices, and/or transducers. In exemplary embodiments, the catheters of the present invention can include a radiopaque distal portion and/or radiopaque markers disposed on a distal portion of the catheter body, such as proximal and distal of the cutting window, on the cam or ramp, so as to allow the user to track the position of the cutter, or the like. The catheters of the present invention will also be particularly useful with ultrasonic transducers, such as an IVUS, of a type which may be deployed linearly within the catheter body or circumferentially on the debulking assembly. Linear deployment will allow viewing along a discrete length of the catheter axis, preferably adjacent to the cutting point, usually over a length in the range from 1 mm to 30 mm, preferably 2 mm to 10 mm. Circumferentially deployed phased arrays may subtend a viewing arc in the range from 5° to 360°, usually from 180° to 360°. For imaging transducers located on cutting blades within a housing or second cutting element, the field of imaging will generally be limited by the dimensions of the aperture. In some cases, however, it might be possible to fabricate all or a portion of the cutter blade/housing out of an ultrasonically translucent material. A more complete description of suitable imaging catheters are described more fully in U.S. patent application Ser. No. 09/378,224, filed Aug. 19, 1999, and entitled "Atherectomy Catheter with Aligned Imager," now U.S. Pat. No. 6,299,622 B1, the complete disclosure of which is incorporated herein by reference. In addition to ultrasonic array transducers, the imaging devices of the present invention may comprise optical coherence tomography devices, such as described in U.S. Pat. No. 5,491,524, as well as Huang et al. (1991) Science 254:1178-1181; Brezinski et al. (1997) Heart 77:397-403; and Brezinski et al (1996) Circulation 93:1206-1213. In some instances, the present invention may also provide optical imaging using optical wave guides and the like.

A catheter constructed in accordance with principles of the present invention comprises a catheter body having a proximal portion and a distal portion. Proximal portion can be coupled to distal portion with a connection assembly to allow pivoting or deflection of distal portion relative to proximal portion. A proximal end of the catheter body can have a handle for manipulation by a user, a luer for connection to an aspiration or fluid delivery channel, or the like.

A debulking assembly, such as a cutter, abrasive member, or the like, is disposed within a lumen of the catheter body. The cutter is typically rotatable within the distal portion about an axis that is parallel to the longitudinal axis of the distal portion of catheter and axially movable along the longitudinal axis. The cutter can access target tissue through a side opening window which is typically large enough to allow the cutter to protrude through and move out of the window a predetermined distance. The cutter is coupled to a cutter driver through a coiled drive shaft. Actuation of a movable actuator or other input device 38 can activate the drive shaft and cutter, move cutter longitudinally over a cam so as to deflect the distal portion and move the cutter out of cutting window. Camming of the cutter can cause the distal portion to pivot or deflect relative to the proximal portion so as to deflect and urge the cutter into the tissue in the body lumen.

In some embodiments, the distal portion of the catheter may be moved to an angled or offset configuration from the longitudinal axis of the proximal portion of the catheter and the cutter. In some embodiments, the cutter can also be deflected off of the axis of the proximal and/or distal portion of the catheter. Moving the distal portion to an angled/offset position may cause a portion of the catheter to urge against a target tissue, may expose the cutter 28 through the window 32 or both, in various embodiments.

In catheters of the present invention, proximal portion is typically relatively flexible and distal portion is typically relatively rigid. Additionally, many embodiments include a flexible distal tip. The flexible proximal portion of the catheter is typically a torque shaft and the distal portion is typically a rigid tubing. The torque shaft facilitates transportation of the catheter body and cutter to the diseased site. The proximal end of the torque shaft is coupled to a proximal handle and the distal end of the torque shaft is attached to the distal, rigid portion of the catheter through the connection assembly. The drive shaft is movably positioned within the torque shaft so as to rotate and axially move within the torque shaft. The drive shaft and torque shaft are sized to allow relative movement of each shaft without interfering with the movement of the other shaft. The catheter body will have the pushability and torqueability such that torquing and pushing of the proximal end will translate motion to the distal portion of the catheter body.

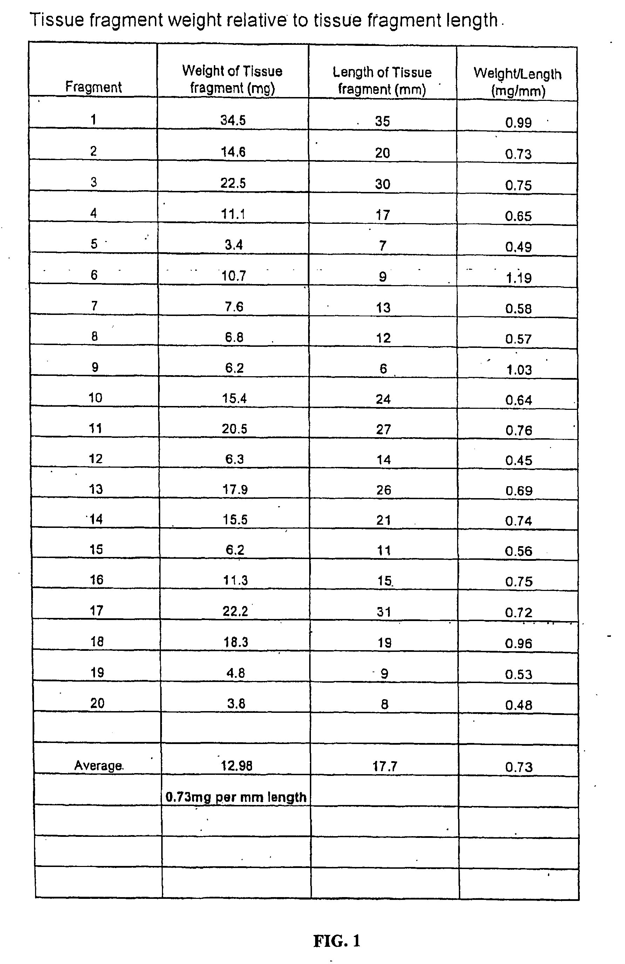

A catheter as in FIG. 1 may have a flexible proximal portion which additionally includes urging means. Urging means may comprise a rigid bent or curved shape towards the distal end of proximal portion, which may help urge the cutter or other debulking apparatus toward a wall of a body lumen to enhance treatment. Such a rigid bend increases the working range of the catheter by allowing the cutter to be urged into a lumen wall across a wider diameter lumen.

In other embodiments, urging means may take many other suitable forms. For example, a similar result to the rigid bend may be achieved by including a rigid distal portion that is not permanently bent but that is more rigid on one side than on the opposite side of catheter body. Thus, when proximal tension is applied to the proximal portion, as when proximal force is applied to the debulking apparatus to expose the cutter through the window, the urging means (i.e., the rigid distal portion of proximal portion) will cause the catheter body to bend toward the less rigid side. The less rigid side will typically be the same side as the window, so that the window and/or the cutter will be urged against a wall of a body lumen by the bend. In still other embodiments, a shaped element may be introduced into catheter body to act as urging means. Any suitable urging means is contemplated.

The catheter includes a connection assembly, a rigid housing, a distal tip that at least partially defines a collection chamber for storing the severed atheromatous material, and a lumen that can receive the guidewire. The distal tip can have a distal opening that is sized to allow an imaging guidewire or conventional guidewire (not shown) to be advanced distally through the tip. In some embodiments, the distal tip may also include a distal guidewire lumen (not shown) for allowing passage of a guidewire. For example, some embodiments may include a distal guidewire lumen having a length of between about 1.0 cm and about 5.0 cm, and preferably between about 2.0 cm and about 3.0 cm. Such a distal guidewire lumen may be used alone or in conjunction with a proximal guidewire lumen located on another, more proximal, portion of the catheter.

In embodiments including a distal guidewire lumen and a proximal guidewire lumen, the distal lumen may be configured to partially telescope within a portion of the proximal guidewire lumen, or vice versa. Such telescoping lumens may be used in embodiments where the distal portion of catheter body is movable relative to the proximal portion. A telescoping lumen may enhance performance of the catheter by allowing a guidewire to be maintained largely within a lumen and to not be exposed within the body lumen being treated. Telescoping lumens may have any suitable diameters and configurations to allow for sliding or otherwise fitting of one lumen within another.

As mentioned above, various embodiments of the invention may allow for deflection of a portion of a catheter, exposure of a tissue debulking assembly through a window, or both. In some embodiments, movement of a tissue debulking assembly causes deflection of a portion of the catheter. In other embodiments, deflection of the catheter may cause a tissue debulking assembly to be exposed through a window on the catheter. In still other embodiments, there may be no causal relationship between deflection of the catheter and exposure of the debulking assembly-i.e., they may be separately caused.

As an example, a ramp or cam may at least partially fit within the distal portion. As will be described in detail below, in some embodiments proximal movement of the cutter over the ramp, causes the deflection of the distal housing and guides cutter out of cutting window. (In other embodiments, a ramp may be used to deflect the distal portion without extending the cutter out of the window.) Attached to the ramp is a housing adaptor that can connect one or more articulation member to the distal tip to create an axis of rotation of the distal portion. The housing adaptor and articulation member allow the distal end of the catheter to pivot and bias against the body lumen. In the illustrated embodiment there are only one housing adaptor and one articulation member, but it should be appreciated that the catheters of the present invention can include, two, three, or more joints (e.g., axis of rotation), if desired. Moreover, the axes of rotation can be parallel or non-parallel with each other.

The catheter can also include a shaft adaptor and collar to couple articulation member to the torque shaft. Shaft adaptor can connect the housing to the torque shaft and collar can be placed over a proximal end of the shaft adaptor and crimped for a secure attachment. It should be appreciated by one of ordinary skill in the art that that while one exemplary catheter of the present invention has the above components that other catheters of the present invention may not include more or fewer of the components described above. For example, some components can be made integral with other components and some components may be left out entirely. Thus, instead of having a separate ramp, the ramp may be integrated with the distal tip to direct the cutter out of the cutting window.

The cutters of the present invention will generally be movable between two or more positions. During advancement through the body lumen, the cutter will generally be in a neutral position in which the cutter is distal of cutting window. In some embodiments, an imaging device can be coupled to cutter so as to image the body lumen through cutting window when cutter is in the neutral position. Once the catheter has reached the target site, the cutter can be moved to an open position in which the cutter is moved to a proximal end of the cutting window and will extend out of the cutting window a distance beyond an outer diameter D of the rigid portion. In most embodiments, in the open position, the cutter will have deflected the distal portion and the cutter's axis of rotation will generally be in line with connection assembly but angled or offset from longitudinal axis of the distal portion of the catheter body. Optionally, in some embodiments, cutter can be moved to a packing position, in which the cutter is moved distally, past the neutral position, so as to pack the severed tissue into a distal collection chamber. It should be appreciated however, that while the exemplary embodiment moves the cutter to the above described positions, in other embodiments of the present invention the cutter can be positioned in other relative positions. For example, instead of having the neutral position distal of the cutting window, the neutral position may be proximal of the window, and the open position may be along the distal end of the cutting window, or the like.

The interaction of the components of the rigid distal portions in one exemplary embodiment of the present invention will be further described. The cutting window is typically a cutout opening in the distal portion. While the size of the cutting window can vary, the cutting window should be long enough to collect tissue and circumferentially wide enough to allow the cutter to move out of the cutting window during cutting, but sized and shaped to not expel emboli into the vasculature. Cams or ramp can be disposed in the distal portion of the catheter body to guide or otherwise pivot the cutter out of the cutting window as the cutter is pulled proximally through tensioning of drive shaft.

A joint is located proximal to the cutting window to provide a pivot point for camming of the distal portion relative to the proximal portion. The bending at a flexible joint is caused by the interaction of cams or ramps with cutter and the tensile force provided through drive shaft. In the exemplary configuration, the joint includes a housing adaptor that is pivotally coupled to the distal rigid portion. The resulting pivoting of the rigid distal portion relative to the proximal portion causes a camming effect which urges the distal housing against the body lumen wall without the use of urging means (e.g., a balloon) that is positioned opposite of the cutting window. Thus, the overall cross sectional size of the catheter bodies can be reduced to allow the catheter to access lesions in smaller body lumens. In exemplary embodiments, the distal housing can deflect off of the axis of the proximal portion of the catheter typically between 0° degrees and 30° degrees, usually between 5° degrees and 20° degrees, and most preferably between 5° degrees and 10° degrees. The angle of deflection relates directly to the urge. Urge, however, does not necessarily relate to force but more to the overall profile of the catheter. For example, the greater the angle of deflection, the larger the profile and the bigger the lumen that can be treated. The ranges were chosen to allow treatment of vessels ranging from less than 2 mm to greater than 3 mm within the limits of mechanical design of the components. It should be appreciated however, that the angles of deflection will vary depending on the size of the body lumen being treated, the size of the catheter, and the like.

In some embodiments, the deflection of the distal portion of the catheter urges the cutter into position such that distal advancement of the entire catheter body can move the rotating cutter through the occlusive material. Because the cutter is moved a distance beyond the outer diameter of the distal portion of the catheter and outside of the cutting window, the user does not have to invaginate the tissue into the cutting window. In some embodiments, for example, the cutter can be moved between about 0.025 mm and about 1.016 mm, and preferably between about 0.025 mm and about 0.64 mm, beyond the outer dimension of the distal housing. It should be appreciated that the cutter excursion directly relates to the depth of cut. The higher the cutter moves out of the cutting window the deeper the cut. The ranges are chosen around efficacy without risk of perforation of the body lumen.

Some embodiments of the catheter include a shuttle mechanism or other similar mechanism for temporarily locking the catheter in a cutting position. Such embodiments generally include a shuttle member and a shuttle stop member. The shuttle stop member is typically disposed at an angle, relative to a longitudinal axis through the catheter. When the cutter is moved into the cutting position in such embodiments, the shuttle member falls into the shuttle stop member and thus locks the debulking apparatus in a cutting position. To unlock the debulking apparatus, the cutter may be advanced forward, distally, to release the shuttle member from the shuttle stop member.

Some embodiments including a shuttle mechanism will also include two joints in catheter body. Thus, catheter body will include a proximal portion, a distal portion and a middle portion. When shuttle mechanism is activated to expose cutter through window, the middle portion may orient itself at an angle, relative to the proximal and distal portions, thus allowing cutter to be urged towards a side of a lumen. Such a two-jointed configuration may provide enhanced performance of the catheter by providing enhanced contact of the cutter with material to be debulked from a body lumen.

Pushing the entire catheter across a lesion removes all or a portion of the lesion from the body lumen. Severed tissue from the lesion is collected by directing it into a collection chamber in the tip via the cutter. Once the catheter and cutter have moved through the lesion, the cutter can be advanced distally to a "part off position" in which the cutter is moved back into the cutting window. The tissue is collected as the severed pieces of tissue are directed into a collection chamber via the distal movement of cutter and catheter. The collection chamber of the tip and distal portion acts as a receptacle for the severed material, to prevent the severed occlusive material from entering the body lumen and possibly causing downstream occlusions. The cutter can interact with the distal edge of the cutting window to part off the tissue and thereafter pack the severed tissue into collection chamber. In exemplary embodiments, the driver motor can be programmed to stop the rotation of the cutter at the part off position so that the cutter can move to a third position and pack the material in the collection chamber in the tip without rotation. Typically, the collection chamber will be large enough to allow multiple cuts to be collected before the device has to be removed from the body lumen. When the collection chamber is full, or at the user's discretion, the device can be removed, emptied and reinserted over the guidewire via a monorail system, as will be described below.

In various embodiments, enhancements to the collection chamber may be included. For example, in some embodiments the collection chamber may be configured to be partially or completely translucent or radiolucent and a portion of the catheter surrounding or adjacent to the window will be radiopaque. This combination of radiolucent collection chamber and radiopaque material adjacent window will enhance the ability of a user to determine how full the collection chamber is, because the fullness of the collection chamber will be directly related to the distance the cutter an advance forward into the collection chamber. By facilitating the assessment of collection chamber filling, these embodiments will reduce the need for manually withdrawing the catheter to examine the collection chamber.

In some embodiments, the collection chamber may connect to the rigid housing by means of interlocking components, which interlock with complementary components on the rigid housing. Such components may resemble a screw-in configuration, for example. Interlocking components will provide a stable connection between the collection chamber and the rigid housing while not increasing the outer diameter of either the chamber or the housing. Generally, collection chamber may be given any suitable configuration, shape or size. For example, collection chamber may have a helical configuration. Alternatively, collection chamber may include a series of circular members, straight linear members, one solid cylindrical or cone-shaped member or the like.

The tip of the catheter can include a lumen having a distal opening and a proximal opening that is sized to receive a guidewire, having a diameter of about 0.356mm (about 0.014 in.), about 0.457mm (about 0.018 in.), about 0.813m (about 0.032 in.) or any other suitable diameter.

The flexible proximal portion of the catheter body may also include a short lumen (e.g., about 12 centimeters in length). In some embodiments, however, the guidewire lumen may be disposed within or outside the flexible proximal portion of the catheter body and run a longer or shorter length, and in fact may run the entire length of the flexible portion of the catheter body. In use, the guidewire can be disposed within lumen on the flexible portion of the catheter body and exit the lumen at a point proximal to the rigid portion of the catheter. The guidewire can then re-enter a proximal opening in the tip lumen and exit through distal opening in the tip lumen. By moving the guidewire outside of the rigid portion of the catheter body, the guidewire will be prevented from tangling with the cutter. Typically, tip lumen will be disposed along a bottom surface of the tip and the lumen will be disposed along a side of the proximal portion of the catheter body so that the guidewire will be in a helical configuration. In various embodiments, the tip lumen and the proximal lumen can have any suitable combination of lengths. For example, in one embodiment the tip lumen may have a length between about 1 cm and about 5 cm, more preferably between about 2 cm and about 3 cm, and the proximal lumen may have a length of between about 8 cm and about 20 cm, more preferably between about 10 cm and about 14 cm.

Some catheters of the present invention include a proximal guidewire lumen coupled with the proximal portion of the catheter body, and a telescoping distal guidewire lumen coupled with either the distal tip, part of the distal portion of the catheter body, or both. The telescoping lumen will typically be attached to the tip or a distal portion, but will also include an unattached portion, which will not be directly attached to any part of the catheter body. This unattached portion (or "free floating lumen") protects a guidewire from contacting a body lumen in which the device is used and also allows the device to be moved more freely, without bending or kinking the guidewire. The telescoping guidewire extends within the proximal lumen at the distal opening of proximal lumen. Again, the telescoping feature allows for movement of the catheter body while preventing or reducing bending of the guidewire. For example, in some embodiments catheter allows for deflection of distal tip and the distal portion of the catheter relative to the proximal portion, for example by movement about a pivot point. Telescoping distal lumen and proximal lumen allow for this movement by allowing distal lumen to telescope within proximal lumen. At the same time, distal lumen protects a guide wire from exposure to a body lumen and/or bodily fluids.

Any suitable configurations and sizes of distal lumen and proximal lumen are contemplated. For example, in one embodiment distal lumen may telescope within proximal lumen by a distance of approximately 1 cm. Furthermore, a telescoping lumen may be longer than distal lumens in other embodiments. For example, telescoping lumen may have a length of between about 2 cm and about 10 cm, and preferably between about 5 cm and about 8 cm. As is apparent from the drawing figures, the outer diameter of telescoping distal lumen is configured to fit within the inner diameter of proximal lumen. Generally, any combination of sizes, lengths, diameters and shapes of distal lumen and proximal lumen may be used, to allow telescoping of one into another.

The catheters of the present invention can include radiopaque markers so as to allow the user to track the position of the catheter under fluoroscopy. For example, as already described, a point or area around or adjacent to the window may be made radiopaque. In other embodiments, the rigid distal portion can be radiopaque and radiopaque markers can be disposed on the flexible shaft. Typically, the markers will be disposed along the top, proximal to the cutting window, and on the bottom of the catheter to let the user know the position of the cutter and cutting window relative to the target site. If desired, the top and bottom markers can be different shaped so as to inform the user of the relative orientation of the catheter in the body lumen. Because the guidewire will form a helix in its transition from lumen to tip lumen, the user will be able to view the top and bottom radiopaque markers without interference from the guidewire. Some embodiments of the catheter can also include a radiopaque cutter stop that is crimped to driveshaft proximal of the cutter that moves with the cutter so as to let the user know when the cutter is in the open position.

The distal portion of the rotatable cutter can include a serrated knife edge or a smooth knife edge and a curved or scooped distal surface. The distal portion may have any suitable diameter or height. In some embodiments, for example, the diameter across the distal portion may be between about 0.1 cm and about 0.2 cm. A proximal portion of the cutter can include a channel that can be coupled to the drive shaft that rotates the cutter. Some embodiments of the cutters can include a bulge or bump that is provided to interact with a stent so as to reduce the interaction of the cutting edge with the stent. In any of the foregoing embodiments, it may be advantageous to construct a serrated knife edge, a smooth knife edge, or a scooped distal surface out of tungsten carbide.

The cutter has a beveled edge, made of tungsten carbide, stainless steel, titanium or any other suitable material. The beveled edge is angled inward, toward the axis of rotation (or center) of the cutter, creating a "negative angle of attack" for the cutter. Such a negative angle of attack may be advantageous in many settings, when one or more layers of material are desired to be debulked from a body lumen without damaging underlying layers of tissue. Occlusive material to be removed from a vessel typically has low compliance and the media of the vessel (ideally to be preserved) has higher compliance. A cutter having a negative angle of attack may be employed to efficiently cut through material of low compliance, while not cutting through media of high compliance, by allowing the high-compliance to stretch over the beveled surface of cutter.

The cutter driver can act as the handle for the user to manipulate the catheters of the present invention as well as a power source. Typically, the cutter drivers of the present invention include a single input device, such as a lever that controls the major operations of the catheter (e.g., axial movement to cause urging, rotation to cause cutting, and axial movement for packing). The cutter driver includes a power source (e.g., batteries), a motor, a microswitch for activating motor, and a connection assembly (not shown) for connecting the drive shaft to the driver motor. In some embodiments, the drive motor can rotate drive shaft between 1,000 rpm and 10,000 rpm or more, if desired.

In use, the catheter will be delivered to the target site with cutter driver unattached and the cutter in the neutral position. The cutter driver can be attached with the urge lever in a neutral position, which indicates that the cutter is closed, but not in a packing position. The user can then move the catheter (and cutter driver unit, if desired) to position the distal portion of the catheter adjacent the target tissue. To activate the rotation of the cutter, the urge lever can be moved proximally from the neutral position to move the cutter proximally and out of cutting window and simultaneously depressing microswitch to activate motor. At the end of the cutting procedure, the user can push urge lever completely forward to a distal position to push the cutter into a packing position. After the urge lever passes the middle of the travel, the microswitch can be released so as to deactivate the cutter before reaching the packing position such that packing can occur without the cutter rotating. It should be appreciated, while the figures illustrate the use of an urge lever or thumb switch as an input device, the present invention can use other type of input devices, such as labeled buttons (e.g., close window, debulk tissue, and pack), or the like.

Advantageously, cutter driver provides an automatic on/off control of the cutter that is keyed to the position of the cutter. Such a configuration frees the user from the complicated task of remembering the sequence of operations to activate and deactivate the rotation and axial movement of the cutter.

While the cutter driver is illustrated as a disposable battery powered unit, it should be appreciated that in other embodiments, the cutter driver can use other power sources to control the cutter driver. It should further be appreciated that other cutter drivers can be used with the present invention. While not preferred, it is possible to have separate controls to control the axial movement of the cutter and the rotation of the cutter.

Some exemplary methods of the present invention will now be described. One method of the present invention comprises delivering a catheter to a target site in the body lumen. A distal portion of the catheter can be deflected relative to a proximal portion of the catheter to expose a tissue debulking device in the catheter. The body lumen can be debulked with the exposed debulking device. Specifically, one specific method comprises advancing a catheter to a target site. A cutter can be rotated and moved out of the cutting window. Preferably, a distal portion of the catheter can be pivoted or deflected so as to position the cutter adjacent the target material. Thereafter, the catheter and the rotating cutter can be moved through the body lumen to remove the target material from the body lumen.

The catheter can be percutaneously advanced through a guide catheter or sheath and over a conventional or imaging guidewire using conventional interventional techniques. The debulking catheter can be advanced over the guidewire and out of the guide catheter to the diseased area. The window will typically be closed (with the cutter or other debulking device in a first, distal position). The catheter will typically have at least one hinge or pivot connection to allow pivoting about one or more axes of rotation to enhance the delivery of the catheter into the tortuous anatomy without dislodging the guide catheter or other sheath. The cutter can be positioned proximal of the lesion. Optionally, a transducer, IVUS, or other imaging assembly can be used to verify the position of the debulking catheter.

Once the position of the catheter is confirmed, the cutter will be retracted proximally and moved out of cutting window to its second, exposed position. In some embodiments, movement of the cutter can deflect the distal portion of the catheter to increase the profile of the catheter at the target site. Movement of the cutter is typically caused by proximal movement of lever and tensioning of drive shaft. Movement of the lever can be scaled to any desired ratio or a direct 1:1 ratio of movement between the handle and cutter. When the cutter is moved proximally it contacts ramp or cam surfaces so as to guide the cutter up and at least partially out of the cutting window. Additionally, as shown by arrow 80, the distal portion of catheter body rotates about the joint to provide an urging force for the cutter (and catheter body) to move toward the diseased area.

Thereafter, the operator can move the entire catheter body through the lesion to dissect the tissue. As the cutter and catheter body are advanced distally through the lesion, tissue that is trapped between the cutting edge and the cutting window is severed from the body lumen. To part off the tissue, the operator can stop pushing the device distally and the cutter can be advanced distally inside the cutting window by advancing the handle. During the distal movement of the cutter, the cutter rides back over the ramps and directs the cutter back inside of the cutting window. Such movement causes the distal portion of the catheter to move in line with the cutter and proximal portion. When the cutter has moved to its distal position, the cutter parts off the severed tissue and urges the severed tissue inside of a collection chamber in the distal tip. Optionally, after the cutter has parted off the tissue, the lever and thus the non-rotating cutter can be advanced distally to pack the tissue into the collection chamber. Use of the cutter to pack the severed tissue will allow the operator multiple specimens to be collected prior to removing the catheter from the body lumen. When it is determined that the collection chamber is full, the catheter can be removed from the body lumen and the collection chamber can be emptied, and the excised tissue may be stored or tested as described above.

In another method of the present invention, an input device is disposed in a first position to position a tissue removal element in a neutral position. The input device is activated to rotate the tissue removal element and to axially move the tissue removal device to an active position. The input device can then be activated again to move the tissue removal element to a packing position. In an exemplary embodiment, the input device is a lever or thumb switch that can be moved to correspond to the movement of a cutting element on the catheter. Thus, as the lever is moved proximally, the cutter is rotated and moved proximally to an open position. When the lever is moved to a distal position, the rotation of the cutter can be stopped and the cutter can be moved distally to pack severed tissue into a collection chamber.

The present invention will further comprise kits including catheters, instructions for use, and packages. Catheters will generally be as described above, and the instruction for use (IFU) will set forth any of the methods described above. Package may be any conventional medical device packaging, including pouches, trays, boxes, tubes, or the like. The instructions for use will usually be printed on a separate piece of paper, but may also be printed in whole or in part on a portion of the packaging.

While all the above is a complete description of the preferred embodiments of the inventions, various alternatives, modifications, and equivalents may be used. For example, while preferred cutters are moved proximally to move the cutter out of the cutting window, alternative embodiments may move the cutter distally to move the cutter out of the cutting window. Additionally, while most embodiments employ a cutter that extends out beyond the outer diameter of the cutting window, it may be possible to incorporate a cutter that stays within the diameter catheter body. Additionally, in some embodiments, the debulking assembly may be exposed through the window without causing a deflection of the distal portion of the catheter. Moreover, instead of having a distal tip that is rotatable relative to the proximal portion of the catheter, the catheter can include a shape memory material such that the catheter forms a jog or a pre-bent shape when it reaches its target area.

## Claims

1. A composition of excised vascular tissue comprising at least one continuous strand of tissue that has been removed from along the axis of the inner surface of a vascular lumen in a human body and preserved, wherein the continuous strand comprises a length of at least 2 cm, a depth of at least 0.1 mm and a volume of at least 0.45 mg/mm of length.

2. The composition of claim 1 which comprises a plurality of continuous strands.

3. The composition of claim 2 wherein the plurality of strands are collected from a single vascular lumen or a single vascular obstruction.

4. The composition of claim 1 wherein the continuous strand has a length selected from the group consisting of 5cm or greater in length, 7cm or greater in length, 10cm or greater in length, and 15cm or greater in length.

5. The composition of claim 1 wherein the continuous strand has a depth selected from the group consisting of at least 0.25 mm, at least 0.33 mm, and at least 0.5 mm, wherein depth is a dimension which is radial to the axis of the lumen.

6. The composition of claim 1 wherein the continuous strand has a volume selected from the group consisting of at least 0.50 mg/mm of length, at least 0.55 mg/mm of length, at least 0.60 mg/mm of length, at least 0.65 mg/mm of length, and at least 0.70 mg/mm of length.

7. A library comprising a plurality of samples of excised vascular tissue according to any one of claims 1 to 6.

8. The library of claim 7 wherein the samples are preserved so as to maintain the continuous strand intact.

9. The library of claim 7 wherein the samples are maintained in a preserving agent, tissue fixative, or test preparation agent selected from the group consisting of saline, heparinized saline, liquid nitrogen, formalin, paraffin, a membrane lysis agent, an RNA preparation agent, and a DNA preparative agent.

10. The library of claim 7 wherein clinical information regarding patients from whom the samples were excised is maintained with the library.

11. A library comprising a plurality of samples of material derived from excised vascular tissue according to any one of claims 1 to 6, wherein the plurality of samples of derived material are selected from the group consisting of DNA, RNA, cDNA, and protein.

12. The library of claim 11 wherein clinical information regarding patients from whom the samples were excised is maintained with the library.

13. An ex vivo method of gathering information about vascular disease in an individual or a population of individuals comprising providing excised vascular tissue removed from a body lumen of one or more individuals wherein the tissue comprises a composition of any of claims 1-6, performing one or more tests on at least a portion of the excised and removed vascular tissue composition, and recording the results of the tests for use in a process selected from the group consisting of identifying, diagnosing, treating, and researching vascular disease.

14. The ex vivo method of claim 13, wherein the tests comprise a test selected from the group consisting of genomic screening, DNA hybridization, RNA hybridization, gene expression analysis, PCR amplification, proteomic testing, drug efficacy screening, determining the presence of one or more DNA markers, determining the presence of one or more RNA markers; determining the presence of one or more protein markers, histological screening, histopathology, cytopathology, cell type analysis, tissue type analysis, and biopsy.

15. The ex vivo method of claim 13, wherein the test comprises analyzing the vascular tissue composition for the presence of DNA, RNA, or a protein marker comprising a moiety selected from the group consisting of a smooth muscle proliferative promoter, a smooth muscle proliferative inhibitor, a cellular marker, an apoptotic marker, a cell cycle protein, a transcriptional factor, a proliferative marker, an endothelial growth factor, an adhesion molecule, a cytokine, a chemokine, a chemokine receptor, an inflammation marker, an extracellular matrix molecule, an interleukin, a growth factor, a glycoprotein, a proteoglycan, a cell-surface marker, a serum marker, and an immune factor.

16. The ex vivo method of claim 13 wherein the vascular tissue has been excised and removed from more than one site in a single vascular lumen of an individual.

17. The ex vivo method of claim 13 wherein the vascular tissue has been excised and removed from more than one vascular lumen in an individual.

18. The ex vivo method of claim 13, wherein the vascular tissue excised and removed from an individual comprises from 1 mg to 2000 mg of vascular tissue.

19. The ex vivo method of claim 13, wherein the vascular tissue excised and removed from a population of individuals comprises on average from 1 mg to 2000 mg of vascular tissue from each individual.

20. The ex vivo method of claim 13, wherein providing excised vascular tissue comprises providing a library according to any one of claims 7 to 10.

21. The ex vivo method of claim 20 further comprising providing a plurality of samples of material derived from vascular tissue selected from the group consisting of DNA, RNA, cDNA, and protein.

## Patentansprüche

1. Zusammensetzung aus herausgeschnittenem Gefäßgewebe, umfassend zumindest einen durchgehenden Gewebestrang, der entlang der Achse der Innenfläche eines Gefäßlumens in einem menschlichen Körper entnommen und konserviert wurde, worin der durchgehende Strang eine Länge von mindestens 2 cm, eine Tiefe von mindestens 0,1 mm und ein Volumen von mindestens 0,45 mg/mm der Länge umfasst.

2. Zusammensetzung nach Anspruch 1, die eine Vielzahl von durchgehenden Strängen umfasst.

3. Zusammensetzung nach Anspruch 2, worin die Vielzahl von Strängen aus einem einzelnen Gefäßlumen oder einer einzelnen Gefäßobstruktion gesammelt werden.

4. Zusammensetzung nach Anspruch 1, worin der durchgehende Strang eine Länge aufweist, die aus der aus 5 cm oder länger, 7 cm oder länger, 10 cm oder länger und 15 cm oder länger bestehenden Gruppe ausgewählt ist.

5. Zusammensetzung nach Anspruch 1, worin der durchgehende Strang eine Tiefe aufweist, die aus der aus mindestens 0,25 mm, mindestens 0,33 mm und mindestens 0,5 mm bestehenden Gruppe ausgewählt ist, worin Tiefe eine Dimension radial zur Achse des Lumens ist.

6. Zusammensetzung nach Anspruch 1, worin der durchgehende Strang ein Volumen aufweist, das aus der aus mindestens 0,50 mg/mm der Länge, mindestens 0,55 mg/mm der Länge, mindestens 0,60 mg/mm der Länge, mindestens 0,65 mg/mm der Länge und mindestens 0,70 mg/mm der Länge bestehenden Gruppe ausgewählt ist.

7. Bibliothek, umfassend eine Vielzahl von Proben von herausgeschnittenem Gefäßgewebe nach einem der Ansprüche 1 bis 6.

8. Bibliothek nach Anspruch 7, worin die Proben konserviert werden, damit der durchgehende Strang intakt bleibt.

9. Bibliothek nach Anspruch 7, worin die Proben in einem Konservierungsmittel, Gewebefixiermittel oder Testpräparationsmittel, das aus der aus Kochsalzlösung, heparinisierter Kochsalzlösung, flüssigem Stickstoff, Formalin, Paraffin, einem Membranlyse-Mittel, einem RNA-Präparationsmittel und einem DNA-Präparationsmittel bestehenden Gruppe ausgewählt ist, aufbewahrt werden.

10. Bibliothek nach Anspruch 7, worin klinische Informationen zu Patienten, von denen die Proben herausgeschnitten wurden, mit der Bibliothek aufbewahrt werden.

11. Bibliothek, umfassend eine Vielzahl von Materialproben aus herausgeschnittenem Gefäßgewebe nach einem der Ansprüche 1 bis 6, worin die Vielzahl von Proben von abgeleitetem Material aus der aus DNA, RNA, cDNA und Protein bestehenden Gruppe ausgewählt sind.

12. Bibliothek nach Anspruch 11, worin klinische Informationen zu Patienten, von denen die Proben herausgeschnitten wurden, mit der Bibliothek aufbewahrt werden.

13. Ex-vivo-Verfahren zur Sammlung von Informationen über Gefäßerkrankungen in einer Person oder einer Gruppe von Personen, umfassend die Bereitstellung von herausgeschnittenem Gefäßgewebe, das aus einem Körperlumen von einer oder mehreren Personen entfernt wurde, worin das Gewebe eine Zusammensetzung nach einem der Ansprüche 1-6 umfasst, die Durchführung von einem oder mehreren Tests an zumindest einem Teil der herausgeschnittenen und entfernten Gefäßgewebezusammensetzung, und die Aufzeichnung der Testergebnisse zur Verwendung in einem Verfahren, das aus der aus Identifizierung, Diagnostizierung, Behandlung und Erforschung von Gefäßerkrankungen bestehenden Gruppe ausgewählt ist.

14. Ex-vivo-Verfahren nach Anspruch 13, worin die Tests einen Test umfassen, der aus der aus Genom-Screening, DNA-Hybridisierung, RNA-Hybridisierung, Genexpressionsanalyse, PCR-Amplifikation, Proteom-Test, Arzneimittelwirksamkeits-Screening, Bestimmung der Anwesenheit von einem oder mehreren DNA-Markern, Bestimmung der Anwesenheit von einem oder mehreren RNA-Markern, Bestimmung der Anwesenheit von einem oder mehreren Proteinmarkern, histologischem Screening, Histopathologie, Zytopathologie, Zelltypanalyse, Gewebetypanalyse und Biopsie bestehenden Gruppe ausgewählt ist.

15. Ex-vivo-Verfahren nach Anspruch 13, worin der Test die Analyse der Gefäßgewebezusammensetzung auf Anwesenheit von DNA, RNA oder einem Proteinmarker umfasst, der einen aus der aus einem Proliferationspromoter der glatten Muskulatur, einem Proliferationsinhibitor der glatten Muskulatur, einem zellulären Marker, einem Apoptose-Marker, einem Zellzyklusprotein, einem Transkriptionsfaktor, einem Proliferationsmarker, einem Endothelwachstumsfaktor, einem Adhäsionsmolekül, einem Zytokin, einem Chemokin, einem Chemokin-Rezeptor, einem Entzündungsmarker, einem extrazellulären Matrixmolekül, einem Interleukin, einem Wachstumsfaktor, einem Glycoprotein, einem Proteoglycan, einem Zelloberflächenmarker, einem Serummarker und einem Immunfaktor bestehenden Gruppe ausgewählten Teil umfasst.

16. Ex-vivo-Verfahren nach Anspruch 13, worin das Gefäßgewebe von mehr als einem Situs in einem einzelnen Gefäßlumen einer Person herausgeschnitten und entfernt wurde.

17. Ex-vivo-Verfahren nach Anspruch 13, worin das Gefäßgewebe von mehr als einem Gefäßlumen einer Person herausgeschnitten und entfernt wurde.

18. Ex-vivo-Verfahren nach Anspruch 13, worin das Gefäßgewebe, das aus einer Person herausgeschnitten und entfernt wurde, 1 mg bis 2000 mg Gefäßgewebe umfasst.

19. Ex-vivo-Verfahren nach Anspruch 13, worin das Gefäßgewebe, das aus einer Gruppe von Personen herausgeschnitten und entfernt wurde, 1 mg bis 2000 mg Gefäßgewebe von jeder Person umfasst.

20. Ex-vivo-Verfahren nach Anspruch 13, worin die Bereitstellung von herausgeschnittenem Gefäßgewebe die Bereitstellung einer Bibliothek nach einem der Ansprüche 7 bis 10 umfasst.

21. Ex-vivo-Verfahren nach Anspruch 20, ferner umfassend die Bereitstellung einer Vielzahl von Materialproben aus Gefäßgewebe, die aus der aus DNA, RNA, cDNA und Protein bestehenden Gruppe ausgewählt sind.

## Revendications

1. Composition de tissu vasculaire excisé comprenant au moins un brin continu de tissu qui a été prélevé le long de l'axe de la surface intérieure d'une lumière vasculaire dans un corps humain et qui a été conservé, dans laquelle le brin continu a une longueur d'au moins 2 cm, une épaisseur d'au moins 0,1 mm et un volume d'au moins 0,45 mg/mm de longueur.

2. Composition suivant la revendication 1, qui comprend une pluralité de brins continus.

3. Composition suivant la revendication 2, dans laquelle la pluralité de brins est collectée à partir d'une lumière vasculaire unique ou d'une obstruction vasculaire unique.

4. Composition suivant la revendication 1, dans laquelle le brin continu a une longueur choisie dans le groupe consistant en une longueur égale ou supérieure à 5 cm, une longueur égale ou supérieure à 7 cm, une longueur égale ou supérieure à 10 cm et une longueur égale ou supérieure à 15 cm.

5. Composition suivant la revendication 1, dans laquelle le brin continu a une épaisseur choisie dans le groupe consistant en au moins 0,25 mm, au moins 0,33 mm et au moins 0,5 mm, l'épaisseur étant une dimension qui est radiale par rapport à l'axe de la lumière.

6. Composition suivant la revendication 1, dans laquelle le brin continu a un volume choisi dans le groupe consistant en au moins 0,50 mg/mm de longueur, au moins 0,55 mg/mm de longueur, au moins 0,60 mg/mm de longueur, au moins 0,65 mg/mm de longueur et au moins 0,70 mg/mm de longueur.

7. Banque comprenant une pluralité d'échantillons de tissu vasculaire excisé suivant l'une quelconque des revendications 1 à 6.

8. Banque suivant la revendication 7, dans laquelle les échantillons sont conservés de manière à maintenir le brin continu intact.

9. Banque suivant la revendication 7, dans laquelle les échantillons sont maintenus dans un agent conservateur, un fixateur de tissus ou un agent de préparation d'essai choisi dans le groupe consistant en une solution saline, une solution saline héparinisée, l'azote liquide, le formol, la paraffine, un agent de lyse des membranes, un agent de préparation d'ARN et un agent de préparation d'ADN.

10. Banque suivant la revendication 7, dans laquelle les informations cliniques concernant des patients desquels les échantillons ont été excisés sont maintenues avec la banque.

11. Banque comprenant une pluralité d'échantillons de matière dérivée d'un tissu vasculaire excisé suivant l'une quelconque des revendications 1 à 6, dans laquelle la pluralité d'échantillons de matière dérivée est choisie dans le groupe consistant en l'ADN, l'ARN, l'ADNc et une protéine.

12. Banque suivant la revendication 11, dans laquelle les informations cliniques concernant des patients desquels les échantillons ont été excisés sont maintenues avec la banque.

13. Procédé ex vivo de réunion d'informations concernant une maladie vasculaire chez un individu ou une population d'individus, comprenant la fourniture d'un tissu vasculaire excisé prélevé à partir de la lumière de l'organisme d'un ou plusieurs individus, le tissu comprenant une composition de l'une quelconque des revendications 1 à 6, la mise en oeuvre d'un ou plusieurs tests sur au moins une partie de la composition de tissu vasculaire excisé et prélevé, et l'enregistrement des résultats des tests pour une utilisation dans un processus choisi dans le groupe consistant en une identification, un diagnostic, un traitement et une recherche de maladie vasculaire.

14. Procédé ex *vivo* suivant la revendication 13, dans lequel les tests comprennent un test choisi dans le groupe consistant en un dépistage génomique, une hybridation d'ADN, une hybridation d'ARN, une analyse d'expression de gène, une amplification par PCR, un test protéomique, une évaluation d'efficacité de substance médicamenteuse, la détermination de la présence d'un ou plusieurs marqueurs d'ADN, la détermination de la présence d'un ou plusieurs marqueurs d'ARN, la détermination de la présence d'un ou plusieurs marqueurs de protéines, un dépistage histologique, une histopathologie, une cytopathologie, une analyse de type cellulaire, une analyse de type tissulaire et une biopsie.

15. Procédé ex *vivo* suivant la revendication 13, dans lequel le test comprend l'analyse de la composition de tissu vasculaire pour la présence d'ADN, d'ARN, ou d'un marqueur de protéine comprenant un groupement choisi dans le groupe consistant en un promoteur de prolifération du tissu musculaire lisse, un inhibiteur de prolifération du tissu musculaire lisse, un marqueur cellulaire, un marqueur d'apoptose, une protéine du cycle cellulaire, un facteur de transcription, un marqueur de prolifération, un facteur de croissance endothélial, une molécule d'adhésion, une cytokine, une chimiokine, un récepteur de chimiokine, un marqueur d'inflammation, une molécule de matrice extracellulaire, une interleukine, un facteur de croissance, une glycoprotéine, un protéoglycane, un marqueur de surface cellulaire, un marqueur sérique et un facteur immunitaire.

16. Procédé ex *vivo* suivant la revendication 13, dans lequel le tissu vasculaire a été excisé et prélevé de plus d'un site dans une lumière vasculaire unique d'un individu.

17. Procédé ex *vivo* suivant la revendication 13, dans lequel le tissu vasculaire a été excisé et prélevé de plus d'une lumière vasculaire chez un individu.

18. Procédé ex *vivo* suivant la revendication 13, dans lequel le tissu vasculaire excisé et prélevé chez un individu comprend 1 mg à 2000 mg de tissu vasculaire.

19. Procédé ex *vivo* suivant la revendication 13, dans lequel le tissu vasculaire excisé et prélevé dans une population d'individus comprend en moyenne 1 mg à 2000 mg de tissu vasculaire provenant de chaque individu.

20. Procédé ex *vivo* suivant la revendication 13, dans lequel la fourniture d'un tissu vasculaire excisé comprend la fourniture d'une banque suivant l'une quelconque des revendications 7 à 10.

21. Procédé ex *vivo* suivant la revendication 20, comprenant en outre la fourniture d'une pluralité d'échantillons de matière dérivée d'un tissu vasculaire choisie dans le groupe consistant en l'ADN, l'ARN, l'ADNc et une protéine.
